(19) Europäisches Patentamt
European Patent Office
Office européen des brevets

(11) EP 1 313 055 A1

(12) **EUROPEAN PATENT APPLICATION**
published in accordance with Art. 158(3) EPC

(43) Date of publication:
**21.05.2003 Bulletin 2003/21**

(21) Application number: **01934523.0**

(22) Date of filing: **04.06.2001**

(51) Int Cl.⁷: **G06F 19/00**, C12N 15/00

(86) International application number:
**PCT/JP01/04697**

(87) International publication number:
**WO 02/001477 (03.01.2002 Gazette 2002/01)**

(84) Designated Contracting States:
**AT BE CH CY DE DK ES FI FR GB GR IE IT LI LU MC NL PT SE TR**
Designated Extension States:
**AL LT LV MK RO SI**

(30) Priority: **28.06.2000 JP 2000193680**
**01.02.2001 JP 2001024990**

(71) Applicant: **Konishi, Tomokazu**
**Akita-shi, Akita 010-0877 (JP)**

(72) Inventor: **Konishi, Tomokazu**
**Akita-shi, Akita 010-0877 (JP)**

(74) Representative: **Zimmermann, Gerd Heinrich**
**Zimmermann & Partner,**
**P.O. Box 33 09 20**
**80069 München (DE)**

(54) **METHOD FOR PROCESSING GENE EXPRESSION DATA, AND PROCESSING PROGRAMS**

(57) A background computing section 32 of an analyzing apparatus 10 computes such a background value that a normal probability graph, based on a cumulative frequency ratio of subtracted values obtained by subtracting the background value from each of values representative of signal intensities on the spot arranged on a DNA chip, has a predetermined linearity. The logarithmically converted values of the corrected signal intensity values, as subtracted values of the background value from the values representative of signal intensities, assume a normal distribution. Accordingly, by standardizing this, it is possible to compare the data measured from the same kind of DNA chips or different kinds of DNA chips.

FIG. 1

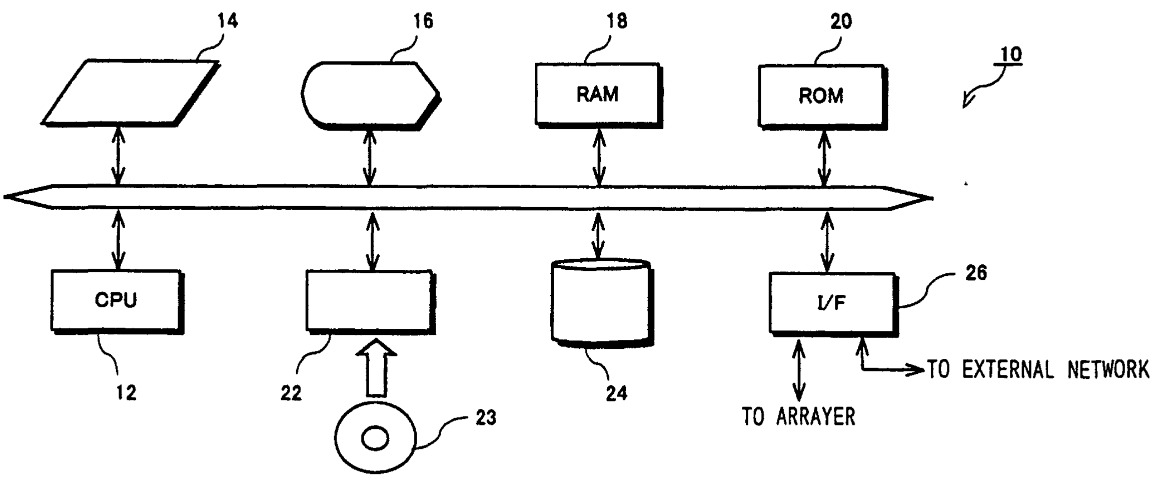


EP 1 313 055 A1

## Description

Technical Field to which the Invention Belongs

The present invention relates to a technique for statistically analyzing the gene expression data acquired from a DNA chip fixed with a multiplicity of genes as spots.

Background of Art

The DNA chip is a fixation of a plurality of genes as different spots on a substrate of a slide glass or the like. For example, a micro-array is fixed with several thousands to several tens of thousands of genes as a target. The target utilizes a single-stranded DNA or mRNA.

The DNA chip substrate can utilize a variety of ones capable of sustaining a nucleic acid thereon, including a plate of a glass or the like processed with a coating in variety, a film of nylon or nitrocellulose, a hollow thread, a semiconductor material, a metal material and an organic substance. Meanwhile, the target can utilize a replication of the entire or a part of cDNA, a replication of a part of genome DNA, synthetic DNA and/or synthetic RNA. For fixing a target on a substrate, there are known a technique of synthesizing an oligo-DNA on a glass plate by a photolithography process and a technique of putting a target on a substrate by the utilization of a spotter or the like.

The DNA chip like this is hybridized by a DNA or RNA (subject of analysis) put with a fluorescent label, for example. The subject of analysis complementary to the target forms double stranding. Because the subject of analysis is put with the fluorescent label, the image data the DNA chip has been manipulated can be acquired, after hybridization, by a fluorescent scanner. On the basis of the image data acquired in this manner, it is possible to know as to whether double stranding is formed in a spot described in any one term. More specifically, the obtained image is displayed with a spot derived from each DNA, as a result of the hybridization. Consequently, by integrating the signal intensities on a predetermined area including a spot position, it is possible to obtain array data constituted by values representative of signal intensities on each spot.

For example, with a micro-array fixed with several thousands to several tens of thousands of targets, array data representative of a multiplicity of gene expressions can be obtained by a once experimental manipulation. As a result of this, when measuring an increase or decrease in the data of a certain one gene expression, it is a general practice to compute, as a subject thereof, an average of the data representative of a multiplicity of gene expressions (values representative of signal intensities) thereby standardizing the data on the basis thereof. More specifically, the data is standardized prior to comparing the expression data of each experiment. For example, there is a disclosure on one example of the standardization in "Normalization strategies for cDNA microarrays (Nucleic Acids Research (2000) Vol.28 No.10)" by Johhanes Schuchhardt et al.

The acquired data is non-parametric in probability distribution. However, as disclosed in "Chasing the dream: plant EST microarrays (Current Opinion in Plant Biology (2000) Vol.3 pp108-116)" by Todd Richmond et al. for example, used is Z-standard, t-standard or a technique of dividing the integration value of signal intensities on each spot by an arithmetic mean of the entire numerals, in order to standardize the acquired data.

Because these are not a non-parametric approach, there has been a problem that such standardization conspicuously impairs the accuracy of data.

Meanwhile, the array data based on an image acquired by a fluorescent scanner contains a background component without exception. This results from the background signal intensity existing in the image data entirety and the nature not always coincident between a measuring range and an actual spot size and form. Accordingly, for correct analysis, it is important to subtract a background component from a numeral of acquired image data, thereby acquiring data having a true signal value. This is true for the array data acquired by another approach, e.g. electric signal detection or radioactive ray detection.

It is a conventional practice to deduce a background component by determining a mean or median value per pixel on the basis of a numeral representative of a signal intensity on a particular spot or non-spotted area and multiplying this value by the number of pixels of a measurement region.

Otherwise, there is also known a technique to deduce a background component from a value at around an outside of a measuring range, as proposed in "ScanAlyze User Manual (http://rana.lbl.gov/EisenSoftware.htm)" by Michael Eisen.

However, in the above conventional correction method, there is change in the background deduced value due to a difference in a region of a spot or image utilized in background value calculation. Namely, there is a possibility that various background values be deduced from the difference, posing a problem that it is impossible to determine which one is proper. In particular, there has been a case the background value increases in difference at between a DNA spotted region and a region not so done.

It is an object of the present invention to provide a technique that the gene expression data acquired from a DNA chip is to be compared with the data of from another DNA chip and wherein proper, statistic analysis is possible to conduct.

Disclosure of the Invention

The present inventor has found a fact that the logarithm values of the data obtained from a DNA chip (data representative of an amount of light emission due to gene expression) assumes a normal distribution. Consequently, by taking logarithm values on the values and hence by logarithmically converting the values representative of signal intensities on each spot and standardizing (e.g. z-standardizing) the same, it is possible to correctly compare the results of different experiments or the experiment results in the same kind. Also, because of storing logarithm and standardized values or utilizing these values during comparison operating, data amount can be made conspicuously small.

More specifically, the object of the invention is to be achieved by a data processing method for processing array data constituted by values representative of signal intensities on each spot arranged on a DNA chip by hybridization of the DNA chip to acquire data to be analyzed, the data processing method comprising: a step of acquiring the array data; a step of logarithmically converting the values representative of signal intensities on each spot constituting the array data; and a step of generating converted data arranged with the logarithmically converted values correspondingly to the spot of the DNA chip.

According to the invention, the group of logarithmically converted values, assuming a normal distribution, is suited in comparing experiment results or analyzing an experiment result using a DNA chip.

In a preferred embodiment, further comprised are a step of scanning the logarithmically converted values to specify a median thereof, and a step of subtracting the median from each value, thereby generating converted data comprising values the median has been subtracted.

The converted data thus obtained is subtracted with the data, as a subject of comparison, subjected to the similar process, making possible to express, in ratio, a comparison result on each spot.

In another embodiment, further comprised is a step of z-standardizing the logarithmically converted values to compute standardized values, thereby generating converted data comprising standardized values.

The converted data thus obtained is subtracted from the data, as a subject of comparison, subjected to the similar process, making possible to express, in a difference, a comparison result on each spot.

Meanwhile, the invention is based on the finding that the data obtained from a DNA chip assumes a logarithmic normal distribution, as in the foregoing. In the invention, it has been made possible to determine more suitable background value. Particularly, conventionally, because of a difference in an area of a spot or image utilized in value computation, the background value has varied to make it impossible to determine which value is proper. The present inventor has found a fact that such correction values as providing a logarithmic normal distribution are proper, on the basis of the finding that the values representative of signal intensities on a spot of a DNA chip assume a logarithmic normal distribution.

In a more preferred embodiment of the invention, further comprised is a step of computing such a background value that a normal probability graph based on a cumulative frequency ratio of subtracted values obtained by subtracting a background value from each of the values representative of signal intensities has a predetermined linearity, the values obtained by subtracting the background value from each of the values representative of signal intensities being rendered as a subject of logarithmic conversion. Incidentally, the background value can take any of positive and negative values. Also, it is possible to consider a case that this value is 0.

In the above embodiment, the step of computing a background' value desirably has a step of specifying a minimum value of the values representative of signal intensities, a step of setting a predetermined range including the minimum value, a step of dividing the predetermined range by a predetermined number to compute, as background value candidate, an upper limit value, a lower limit value and a predetermined number of median values obtained by partitioning, a step of subtracting, for each of the background value candidates, a background candidate value from each of the values representative of signal intensities to compute a subtracted value thereby determining a normal probability graph based on the subtracted values, and a step of specifying a background candidate utilized in an excellent linearity of among the normal probability graph, whereby a range of the upper limit value and lower limit value is changed to a satisfactory in a linearity concerning the specified background candidate, again repeating to compute a background value candidate, to compute a normal probability graph and to specify a background candidate. The step of representing the predetermined linearity can be realized by carrying out a chi-square test.

Meanwhile, in another referred embodiment, the step of computing a background value has a step of making reference to the values representative of signal intensities to specify values in a predetermined percentile of 2 or more, and a step of deducing a background value on the basis of the specified values of 2 or more. Herein, the range of values representative of signal intensities to be utilized is desirably an effective measuring range, i.e. range holding for a linearity of signal response.

More preferably, the step of computing a background value has a step of determining a lower quartile LQ, an upper quartile UQ and a median M from the values representative of signal intensities, and a step of determining

$$x = (UQ * LQ - M^2) / (UQ + LQ - 2M)$$

wherein, x = 0 when UQ + DQ - 2M = 0 to take a determined x as a background value.

Meanwhile, in another embodiment of the invention, correction can be made for a deviation in a vertical direction, a horizontal direction or a radial form of an image hue of the DNA chip.

This embodiment has a step of classifying the spots into a plurality of groups according to an arrangement of the spots of the DNA chip, a step of specifying, for each of the groups, from logarithmically converted values concerning the spot constituting the group, a median thereof, and a step of subtracting the median from each of the logarithmically converted values.

Otherwise, may be comprised a step of classifying the spots into a plurality of groups according to an arrangement of the spots of the DNA chip; a step of specifying, for each of the groups, from values representative of signal intensities concerning spots constituting the group, a median thereof, and a step of dividing each of the values representative of signal intensities by the median.

In the above embodiment, the step of classification may have a step of acquiring, based on each of one or a plurality of columns or one or a plurality of rows, logarithm values concerning the spots included in the column or row in the DNA chip.

Furthermore, in another embodiment, a method of comparing values representative of signal intensities on a plurality of spots by utilizing the data processing method has a step of dividing a converted data value related to one spot by a converted data value related to another spot.

Furthermore, in another embodiment, a method of comparing values representative of signal intensities on a plurality of spots by utilizing the data processing method has a step of comparing a difference value between one standardized value and another standardized value. Herein, it is desired to further comprise computing an exponentiation of a predetermined number on the difference value.

Meanwhile, the object of the invention is to be achieved also by a data processing program for a computer to execute a data processing method of processing array data constituted by values representative of signal intensities on each spot arranged on a DNA chip by hybridization of the DNA chip to acquire data to be analyzed, the data processing program for a computer to execute comprising: a step of acquiring the array data; a step of logarithmically converting the values representative of signal intensities on each spot constituting the array data; and a step of generating converted data arranged with the logarithmically converted values correspondingly to the spot of the DNA chip.

The substrate of a DNA chip can utilize an arbitrary one capable of sustaining a nucleic acid on a surface, including a plate of a glass or the like processed with a coating in variety, a film of nylon or nitrocellulose, a hollow thread, a semiconductor material, a metal material and an organic substance. Also, the DNA chip is arranged thereon, as a target, with a replication of the entire or a part of cDNA, a replication of a part of genome DNA, synthetic DNA and/or synthetic RNA.

Meanwhile, to fabricate a chip, there are included a technique that a nucleic acid is prepared and this is arranged on the substrate by absorption, bond due to static electricity or covalent bond, and a technique that a nucleic acid is synthesized on a substrate. Detecting a signal representative of a signal intensity includes an electric technique utilizing a semiconductor chip and a technique to detect fluorescence or radioactive rays.

The invention is applicable also to the array data from a DNA chip formed with any target on any of the foregoing substrates. Also, application is possible for the array data acquired by using any of the techniques.

Incidentally, in the present specification, the DNA chip includes an arbitrary one arranged with a nucleic acid on a substrate, such as an RNA chip forming RNA on a substrate, a micro-array, a macro-array, a dot-blot or a reversed nozan.

## Brief Description of the Drawings

Fig. 1 is a hardware configuration diagram of an analyzing apparatus according to a first embodiment of the present invention.

Fig. 2 is a block diagram showing an essential part of the analyzing apparatus of the embodiment.

Fig. 3 is a flowchart showing a process to be executed in a background computing section of the analyzing apparatus of the embodiment.

Fig. 4 is a flowchart showing a process to be executed in the background computing section of the analyzing apparatus of the embodiment.

Fig. 5A is a diagram explaining logarithmic conversion and Fig. 5B is a flowchart showing a process to be executed in a conversion processing section and standardization processing section.

Fig. 6 is a histogram of the data acquired by a technique according to the embodiment.

Fig. 7 is a histogram of the data acquired by a conventional technique for comparison.

Fig. 8 is a figure of plotting, on a graph, the values of after standardization obtained in each experiment by carrying out a process of the embodiment on a set of array data acquired from an experiment in different temperature environments.

Fig. 9 is a graph showing, for comparison, a result of standardization carried out based on a frequency distribution shown in Fig. 7.

Figs. 10A to 10D are respectively graphs prepared based on corrected values according to a correction method according to the embodiment.

Figs. 11A to 11D are respectively graphs prepared based on corrected values according to a conventional correction method.

Figs. 12A and 12B are respectively block diagrams showing an essential part of an analyzing apparatus of a second and third embodiment.

Fig. 13 is a flowchart showing a process to be executed in a deviation-correction operating section of the second embodiment.

Fig. 14 is a flowchart showing a process to be executed in a deviation-correction operating section of the third embodiment.

Figs. 15A and 15B are respectively scatter diagrams comparing between the data subjected to deviation-correction according to the embodiment and the data not subjected to deviation-correction.

Preferred Embodiment for Carrying Out the Invention

Hereunder, embodiments of the present invention will be explained with reference to the attached drawings. Fig. 1 is a hardware configuration diagram of an analyzing apparatus according to a first embodiment of the invention. As shown in Fig. 1, an analyzing apparatus 10 has a CPU 12, an input unit 14 such as a mouse or a keyboard, a display unit 16 configured by a CRT or the like, a RAM (Random Access Memory) 18, a ROM (Read Only Memory) 20, a portable storage-medium driver 22 for access to a portable storage medium 23 of CD-ROM, DVD-ROM or the like, a hard disk unit 24, and an interface (I/F) 26 for controlling data exchange with the external. As can be understood from Fig. 1, a personal computer or the like can be utilized as the analyzing apparatus 10 of this embodiment.

The I/F 26 is connected to a reader or scanner to measure a light emission amount on a spot of a hybridized DNA chip and generate data on the basis of a measured light emission amount, and to a communication circuit. The communication circuit is further connected to an external network (e.g. the Internet).

In this embodiment, the portable storage medium 23 is stored with a program to receive data from the reader or scanner and carry out a required data conversion process, referred later, on the data and a program to analyze the processed data. Consequently, the portable storage-medium driver 22 reads the above program out of the portable storage medium 23 and stores it to the hard disk unit 24. By starting this up, the personal computer is allowed to operate as an analysis apparatus 10. Otherwise, the programs may be downloaded via an external network such as the Internet.

Fig. 2 is a block diagram showing an essential part of the analyzing apparatus 10 of this embodiment. In Fig. 2, there is shown a constituent part showing a required data conversion process on data. More specifically, the analyzing apparatus 10 has a data buffer 30, a background computing section 32 to compute a background on the basis of the data (base data) temporarily stored in the data buffer 30, a correction operating section 34 to correct data by the use of a background value obtained in the background computing section 32, a data converting section 36 to carry out a conversion, referred later, on corrected data, and a standardization processing section 38 to standardize the data to which data conversion has been done.

The data buffer 30 is realized its function by the RAM 18 or, in some cases, by the hard disk unit 24. The data buffer temporarily stores the data representative of a light emission amount on each spot transferred from the reader or scanner, or the data representative of a light emission amount on each spot having been transferred from the reader or scanner and previously stored in a predetermined area of the hard disk unit 24. Also, the data buffer can temporarily store the data standardized by the standardization processing section 38.

From the reader or scanner is outputted, as array data, an integration of spot-based signal intensities due to shooting a DNA chip by a CCD camera. Otherwise, there is a case that a background value is computed as a pre-process on the basis of the image data of an image shot by the CCD camera to subtract the background value from a signal intensity on each pixel so that, from the image data having been background-corrected as the pre-process, spot-based signal intensities are integrated and outputted as array data. In this embodiment, any can be utilized of un-processed array data and pre-processed (background-corrected) data. Incidentally, in the present specification, the data cumulative of the spot-based signals transferred from the reader or scanner is referred to as array data, or base data in a meaning of data as a basis to carry out a background process of the embodiment.

Explanation is made on a process to be executed in the background computing section 32 of the analyzing apparatus 10, with reference to Figs. 3 and 4.

The background computing section 32, 'at first, scans the integration values of spot-based signal intensities (spot integration values) contained in the array data stored in the data buffer, to acquire a minimum value thereof (step 301). Next, the background computing section 32 determines whether the acquired minimum value is zero (0) or not (step 302). In the case of zero (Yes in the step 302), a candidate value "A" is set at "-100" while a candidate value "B" at "100" (step 303). The fact the spot integration value is "0" means an absence of light emission amount (image displayed black). In actual, the fact the integration value of spot signal intensities is "0" means an inappropriate measurement or an already subtracted background value by another approach. In such a case, a predetermined negative value is taken as a candidate value "A" and a predetermined positive value is as a candidate value "B", to have a start point in finding a proper background value.

Contrary to this, in the case of a determination of No in the step 302, the background computing section 32 sets the candidate value "A" at a half of the minimum value (1/2 • (minimum value)) and the candidate value "B" at twice the minimum value (2 · (minimum value)) (step 304). Incidentally, the candidate value "A" means an upper limit value to be utilized in a process to specify a background value while the candidate value "B" means a lower limit value.

Next, the background computing section 32 divides between the candidate value "A" and the candidate value "B" equally into nine, to acquire further eight candidate values (step 305). For example, in case the minimum value is "20", the candidate value "A" is "10" and the candidate value "B" is "40", then the following values are candidate values.

candidate value "$C_1$" = 13.33

candidate value "$C_2$" = 16.67

candidate value "$C_3$" = 20.00

candidate value "$C_4$" = 23.33

candidate value "$C_5$" = 26.67

candidate value "$C_6$" = 30.00

candidate value "$C_7$" = 33.33

candidate value "$C_8$" = 36.67

In this manner, totally ten candidate values are obtained.

Furthermore, in the base data (i.e. array data), each candidate value is subtracted from the spot integration value. This obtains 10 sets of spot integration value groups related to the candidate values. The spot integration value groups are respectively referred to as correction data candidates.

Next, the background computing section 32 obtains logarithm values of the spot integration values constituting each correction data candidate, to acquire a cumulative frequency ratio thereof (step 307). The cumulative frequency ratio is plotted to make ten normal probability graphs (step 308). The background computing section 32 tests for a graph linearity on the respective normal probability graphs by the use of a method of least square (step 309). A candidate value utilized is specified for the one most preferred in linearity of among the ten normal probability graphs (step 401). If this is the candidate "A" (Yes in step 402), the background computing section 32 sets one-third of the candidate "A" (1/3 • (candidate "A")) as a new candidate value "A" and one-third of the candidate "B" (1/3 • (candidate "B")) as a new candidate value "B" (step 403). Herein, the range for finding a candidate value is shifted (a little) to the lower.

On the other hand, in the case the relevant candidate value is the candidate "B" (Yes in step 404), the background computing section 32 sets three times the candidate "A" (3 • (candidate "A")) as a new candidate value "A" and three times the candidate "B" (3 • (candidate "B")) as a new candidate value "B" (step 405) . Thismeans that the range for finding a candidate value has been shifted to the upper.

Furthermore, in the case the candidate value is not the candidate value "A" or candidate value "B" (No in step 404 and No in step 405), it is further determined whether the obtained normal probability graph has a satisfactory linearity or not (step 406). This embodiment conducts a chi-square test with a significant level of 5% in order to determine a "satisfactory linearity". However, this is not limitative but other approach may be utilized. The operator may determine that a linearity is satisfactory at his or her own determination.

In the case of a determination of No in step 406, the candidate value "A" is set to an adjacent one to a specified candidate value of among the smaller candidate values than the candidate value specified in the step 401 (step 407). Also, the candidate value "B" is set to an adjacent one to a specified candidate value of among the greater candidate values than the candidate value specified in the step 401 (step 408).

For example, in the candidate value "$C_1$" to candidate value "$C_8$" listed above, the candidate value "$C_3$" was specified in the step 401. The relevant candidate value is assumed that a satisfactory linearity is not obtained on a normal probability graph utilizing the current value from the spot integration value group. In this case, the candidate value "$C_2$" is a new candidate value "A" while the candidate value "$C_5$" is a new candidate value "B". Namely, in the steps 407 and 408, the range for finding a candidate value is narrowed in order to find a more suited candidate value.

In case a new candidate value "A" and candidate value "B" are obtained in step 403, step 405 or steps 407 and 408, the process of step 305 and the subsequent is repeated. Contrary to this, in the case the normal probability graph has a satisfactory linearity (Yes in step 406), the candidate value utilized in obtaining this normal probability graph is determined a background value (step 409).

Next, the correction operating section 34 computes subtractions of the background value acquired in the step 409 from each signal cumulative value constituting the array data. Note that, in this embodiment, one set of the ten sets of correction data candidates is the subtraction of the background value from each signal cumulative value in the step 306 executed immediately before obtaining finally the background value. Accordingly, in case such a correction data candidate is stored in the data buffer 30, the correction operating section 34 may read a proper data candidate from the data buffer 30 without carrying out a new operation.

The correction data, configured by the signal cumulative values (correction signal cumulative values) the background value has been subtracted, is conveyed to the conversion processing section 36. The conversion processing section 36 logarithmically converts the correction signal cumulative value to obtain a converted signal cumulative value. Fig. 5A is a figure showing a process scheme to be executed by the conversion processing section 36. As shown in Fig. 5A, correction signal cumulative values "$a_{ij}$" are taken, in order, out of a table-formatted data region 30-1 comprising the correction signal cumulative values the background value has been subtracted, and subjected to logarithmic conversion (see reference 500). The values subjected to logarithmic conversion (logarithmically converted values) "$\ln a_{ij}$" are arranged in the corresponding positions in a cdnverted table-formatted data region 30-2.

Incidentally, in step 306 and step 307 of Fig. 3, computed are correction data candidates and logarithmically converted values of the correction signal cumulative values constituting the correction data candidate. Consequently, in case logarithmically converted values related to a selected background value are stored in the data buffer 30, the conversion processing section 36 satisfactorily reads out data in the data buffer without the necessity to carry out logarithmic conversion on the correction signal cumulative value.

In case a logarithmically-converted value group is obtained in this manner, the process shown in Fig. 5B is executed by the conversion processing section 36 and standardization processing section 38.

Herein, the conversion processing section 36 sets the number of ranks and a class width (step 501) to prepare a frequency distribution table (step 502). In this embodiment, a graph based on the frequency distribution table is generated. This is displayed on a screen of the display unit 16 (step 503). The step 503 and the step 505 mentioned later are provided in order to verify a correctness of the approach of this embodiment.

Fig. 6 is an example of an image obtained in this manner. In Fig. 6, the horizontal axis represents a logarithmic conversion of correction signal cumulative value (logarithmically converted value) while the vertical axis represents a frequency thereof. In the example shown in Fig. 6, random selection is made avoiding duplication from a rice-plant cDNA library, to utilize a micro-array (cDNA chip) spotted on a matrix having 32 $\times$ 10 per pin. In the micro-array, the total number of effective spots was 1157. In hybridization target preparation, poly(A)RNA derived from rice-plant vagina was used as a mold to synthesize cDNA labeled with cy5. Meanwhile, the result of hybridization was acquired as an image by the use of ArrayScanner V4. 4 (made by Moloecular Dynamics) . This was digitized by using Array Vision program (made by Moloecular Dynamics).

Meanwhile, Fig. 6 shows the ranks including an arithmetic means by a blacked graph. Fig. 7 shows a histogram based on the same array data, for comparison sake. From Figs. 6 and 7, it would be understood that array data itself is non-parametric whereas the logarithmically converted value obtained from the array data is parametric.

In this embodiment, the standardization processing section 38 further makes z-standardization (normalization) on the data on the basis of an acquired frequency distribution in order to enable for data comparison (step 504). This can make common the graph horizontal-and-vertical axes regardless of array data kind or the like, thus enabling comparison between ones of those of data.

Fig. 8 is a plotting, on one graph, of post-normalization values obtained in each experiment by subjecting the set of array data acquired from the experiments different in temperature environment to the process of this embodiment by utilizing the micro-array (cDNA chip) utilized in obtaining the histogram of Fig. 6.

In Fig. 8, the same form of dots (e.g. ×-marks, Δ-marks) represent those acquired in the same experiment. As shown in Fig. 8, the dots on the graph are nearly overlapped with a standard distribution curve shown by a hairline, showing an appropriateness in using a parametric approach. The bold broken line of Fig. 9 is a graph showing, for comparison, a result of normalization carried out based on the frequency distribution shown in Fig. 7. The hairline of Fig. 9 shows a standard distribution curve. From Fig. 9, it would be understood that a parametric approach is not suited on such a form of histogram.

In this manner, by the standardization processing section 38, the data z-standardized (standardized data) is stored to the data buffer 30. By using standardized data, various analyses, experiment verification and the like are possible to conduct.

In this manner, according to this embodiment, it has been found that a normal distribution is given by the logarithm values of the integration values representing signal intensities on each spot of a DNA chip, based on which finding a background value is computed. Also, from'the finding, the integration values (or background-corrected integration values) are logarithmically converted and subjected to z-standardization, thereby acquiring standardized data. Accordingly, by utilizing the standardized data, it is possible to easily compare experiment results in different kind or the same kind thereby enabling to carry out experiment verification.

Meanwhile, according to the background correction of this embodiment, it is possible to conspicuously reduce a cut-out operation in image data. Conventionally, the spot region in an image shot by a CCD camera is specified in a certain extent by the software incorporated in a reader or scanner. However, in actual, there are often cases that a spot and a region to be cut out for integrating signal intensity values are not properly overlapped together. Accordingly, there has been a necessity for a researcher to make reference to an image and set such a circular region as overlapped with the spot. This has been an operation requiring several hours to one day. In case the background correction of this embodiment is utilized, the array may be partitioned into a matrix form such that the cells are equal in area and the spot is included in each cell, thereby acquiring a signal-intensity integration value in the relevant cell. Otherwise, in such a circular region that the areas are equal and a spot is involved (i.e. greater than the spot), integration may be made of the values representing each of the signal intensities at and around the spot.

This is realized from the fact that the background value is to be considered constant in each cell or each circular region provided that the area is the same and the fact that such a background value is computed that the logarithm values of corrected signal integration values are in a normal distribution.

Incidentally, showing and explanation is made below on a correction result with utilizing a background value according to this embodiment. The present applicant has randomly extracted four out of a plurality of ones of expression data based on a plurality of organism species opened to the public in Stanford University (open to the public in http://genome-www4.stanford.edu/MicroArray/SMD and the outline of the opened data is reported also in "The Microarray Database (Nucleic Acids Research 29, pp152 - 155 (2001) by Gavin Sherlock et al.). Herein, utilized are Experiment No. 5733, Experiment No. 1300, Experiment No. 5745 and Experiment No. 7428. Channel 2 was utilized for Experiment No. 7428 while Channel-1 data was utilized for the other experiments.

In each experiment, the logarithm values of the values were z-standardized and then ranked to plot the obtained values on a normal probability paper. Figs. 10A - 10D are respectively graphs obtained from the values corrected, according to the correction method of this embodiment (see Figs. 3 and 4), in respect of Experiment No. 5733, Experiment No. 1300, Experiment No. 5745 and Experiment No. 7428 (channel 2). From these figures, the graphs have sufficient linearities. This shows that the standardized result is in a normal distribution.

Figs. 11A - 11D are graphs obtained by plotting, on a normal probability paper, the values obtained through z-standardization and then ranking of the logarithm values of the values, in each experiment similarly, from the correction based on the conventional correction method (the foregoing approach by Michael Eisen). From these figures, there is shown that the graph has a low linearity and not sufficiently corrected except for Channel 2 of Experiment No. 7438.

Next, explanation is made on a second embodiment of the invention. In the second embodiment, correction can be made for data deviation resulting from unevenness in hybridization due to a problem of planarity in a microarray substrate material or the like.

In an image obtained from a microchip of after hybridization, there is a case that, for example, a central region is rather whity to be blackened as the periphery is neared. Otherwise, there is a case that the entire hue is in a gradation form in a left-and-right or upper-and-lower direction. This is caused, for example, due to strain in a glass utilized in an array base.

For this reason, in the second embodiment, provided is an assumption that the median of signal integration values in each column or each row is generally the same in the array provided that hybridization is carried out ideally, to determine a correction constant to the data common to each column or each row. This is utilized to further correct the signal value.

Fig. 12A is a block diagram showing an essential part of an analyzing apparatus according to the second embodiment. In Fig. 12A, those same as the constituent elements shown in Fig. 2 are attached with the same references. As shown in Fig. 12A, the analyzing apparatus of the second embodiment is provided with a deviation-correction operating section 40 between a correction operating section 34 and a conversion processing section 36.

Fig. 13 is a flowchart showing a process to be executed by the deviation-correction operating section 40 of the second embodiment. The deviation-correction operating section 40 acquires a logarithm value group of signal integration values, a background has been subtracted, acquired by the conversion processing section 36.

Next, the deviation-correction operating section 40 classifies the relevant logarithm value group into column-based groups on the basis of the information representative of a micro-array row and column (step 1302). By determining a predetermined correction constant for each group, deviation correction is realized.

On the basis of a logarithm value belonging to a first column (column no. = 1 (see reference 1303)), the deviation-correction operating section 40 specifies its median value (step 1304), and subtracts the median value from each logarithm value to compute a deviation correction value (step 1305). Namely, the median value is a correction constant for deviation correction in the column. The process shown in step 1304 and step 1305 is executed for all the columns in the number of n (see steps 1306 and 1307).

In this manner, the deviation correction group obtained is standardized in the standardization processing section 38. The scatter diagrams, comparing the data deviation-corrected according to the embodiment and the data not deviation-corrected, are respectively shown in Figs. 15A and 15B. Herein, the micro-array utilized the one bonded with two sets of matrixes having 12 grids each having 32 columns by 12 rows by spotting rice-plant cDNA. This micro-array is hybridized by cDNA, derived from rice-plant cultivated cells, labeled with cy5.

Fig. 15B is a scatter diagram based on the data that, by the approach of the first embodiment, a background value was computed for each set so that this is utilized to correct the value and furthermore subjected to logarithmic conversion and standardization. Fig. 15A is a scatter diagram based on the data deviation-corrected by the approach of the second embodiment. In these figures, two hairlines respectively represent $2^{1/2}$ times (root 2 times) and $(1/2)^{1/2}$ times (root (1/2 times) in y-axis value as compared to the x-axis value.

Because the two sets are the same one of hybridization comes from a result of a pair of spots provided on the same array-chip, dots are principally positioned in a linear form of X = Y. Referring to Figs. 15A and 15B, it can be understood that data dispersion is reduced by deviation correction.

In this manner, according to this embodiment, correction can be properly made for the value change resulting from unevenness in hybridization or the like.

Next, explanation is made on a third embodiment. In the third embodiment, modification is made to the deviation correction of the second embodiment. Fig. 12B is a block diagram showing an essential part of an analyzing apparatus according to the third embodiment. In Fig. 12B, those same as the constituent parts shown in Fig. 2 are attached with the same references. In the third embodiment, a deviation-correction operating section 42 is interposed between a data buffer 30 and a background computing section 32, to carry out deviation correction on the signal integration values constituting array data prior to computing a background value.

Fig. 14 is a flowchart showing a process for deviation correction according to the third embodiment. As shown in Fig. 14, the deviation-correction operating section 42, when acquiring a signal integration value group from the data buffer (step 1401), classifies them into column-based groups on the basis of the information representative of a micro-array second and column (step 1402). Next, the deviation-correction operating section 42 specifies, on the basis of an integration value belonging to the first column (column no. = 1 (see reference 1403), its median value (step 1404) and divides each integration value by the median value to compute deviation correction values (step 1405). Namely, also herein, the median value is a correction constant for deviation correction in the column.

The process shown in step 1404 and step 1405 is to be executed for all the columns in the number of n (see steps 1406 and 1407). In this manner, background value computation is carried out in the background computing section 32, for the deviation-correcting value group obtained.

Next, explanation is made on the data comparison according to the invention. In the first to third embodiments, the corrected signal integration values are logarithmically converted to acquire logarithm values. Furthermore, computed are values the logarithm values are standardized (standard values).

By using these standard values, the following comparison is made possible.

According to this embodiment, the above standard values are utilized to make it possible to find a ratio in amount of RNA, i.e. ratio of gene expression. For example, the foregoing ratio can be determined by taking a difference between a standard value on a certain spot and a standard value on another spot, and multiplied thereon by a standard deviation to take an exponentiation of 10 on the value thereof. The difference in gene expression ratio between standard values "1" and "2" concerned with the spot, if using common logarithm, can be quantified as expressed by the following formula.

$$10 ^ \{(2 - 1) * 0.5\} \approx 3.1$$

(where 0.5 is a standard deviation on the logarithm value)

Namely, the difference in ratio can be expressed in a form of (base of logarithm) ^ { (difference in standard values) * (standard deviation on the logarithm value)}.

Such comparison is possible between arbitrary spots, such as between different spots on the same DNA chip or between the spots with the same gene on different DNA chips. By enabling the quantification on spot-to-spot comparison, it is possible to properly grasp as to what gene is expressed in what amount, what gene increases to what extent between experiments, or so.

The invention is not limited to the foregoing embodiments but can be modified in various ways within a scope set forth in the claims. It is needless to say that those are also included in the scope of the invention.

For example, according to the present embodiment, a predetermined range including a minimum value of spot signal intensity is set to compute a background value by try-and-improvement (see Fig. 3). However, this is not limitative. Utilizing a Lower Quartile (LQ), Upper Quartile (UQ) and Median (M) of a value representative of the above signal intensity, robust deduction may be done. After ideal correction, because the quartiles are in symmetric positions about the median, a background value x is given by the following equation.

$$\ln (UQ - x) - \ln (M - x) = \ln (M - x) - \ln (LQ - x)$$

By solving this, we obtain

$$x = (UQ * LQ - M^2) / (UQ + LQ - 2M).$$

wherein, x = 0 when UQ + DQ - 2M = 0

By subtracting this x from a value representative of a signal intensity on each spot (signal integration value), a corrected signal integration value may be acquired.

Otherwise, a background value may be deduced using other percentiles, e.g. an upper quartile (UQ) and a median (M) by a similar way. Furthermore, using much more percentiles, background values x can be determined to acquire a mean value thereof thereby enhancing the accuracy in the above deduction value. Because percentile and z (zeta) score are in a one-to-one correspondence on a normal distribution, a background value x can be determined by establishing and solving an equation similar to the foregoing equation with utilizing a combination of arbitrary two percentiles that a z-score difference is to be made equal.

Furthermore, the range of signal integration values, utilized in computing a background value in this embodiment, may be given a range holding for the linearity in signal response in a system for a series of measurements including hybridization experiments and reader or scanner characteristics.

Meanwhile, although a predetermined range including a minimum value of signal integration values is set in the process shown in Fig. 3, this is not limitative. For example, considering

$$\text{background value / (median of signal integration values) = c (constant),}$$

in order to determine c of

$$\text{background value = c * (median),}$$

a similar process may be executed.

Meanwhile, in the second embodiment and third embodiment, although spots are classified into groups comprising one or a plurality of columns in a micro-array, this is not limitative. It is needless to say that classification may be into one or a plurality of rows. Also, as in the foregoing explanation, there is a case that image hue is in a gradation form in a direction from a peripheral region of an array toward its center. In such a case, the micro-array may be partitioned in a plurality of hollow rectangles forming nested boxes so that the signal integration values on the spots included in each rectangle are made belonging to the same group to compute a deviation-correcting value for each group.

Meanwhile, in the foregoing embodiments, although z-standardization is utilized as standardization, this is not limitative. It is needless to say that other standardizations are also applicable.

Industrial Applicability

The present invention is applicable to various comparisons, such as a comparison of a result of experiment changed in condition on a DNA chip in the same kind or a comparison of a result of experiment on the DNA chips different in kind. For example, the present invention has screened a gene for working during germinating a rice plant at low temperature, out of a group of ten thousand of genes. On this occasion, using a micro-array pasted with independent genetic fragments in ten thousand of kinds, RNAs were taken out of two tissues, for example, of

a) a rice plant germinated at a warm place
b) the one thereof exposed to a low temperature, and then the respective were hybridized. Experiments were conducted twice on each RNA. As a result of respective experiment results, obtained is a marshal of numerals (relative values) in the number of ten thousand. It is a current situation that there is no appropriate method for comparing the marshal of numerals. According to the embodiment of the invention, standardized data was obtained on the basis of the experiment result due to the respective conditions of a) and b). By subtracting a value of the corresponding spot, an mRNA was found which is to increase or decrease in cumulative amount upon being exposed to a low temperature, thus screening out an objective gene.

Furthermore, with standardized data, comparison is possible beyond the difference in DNA chips, the difference in organism species or the like. For example, in the above experiment a), a group of protein genes, called "thermo-shock protein", was detected in an amount of 2 - 3 standard units. However, these proteins are to be detected in an amount of 0 (zero) standard unit at all times from a tissue of a plant, called *shiroinunazuna*, raised in a normal way. This difference was in a degree not to be explained by accidentality or species-to-species difference. This result showed that the experiment system of a) was "excessively hot". Accordingly, it was possible to find that screening is to be conducted more accurately by cooling a little the first experiment system.

In this manner, according to the invention, application is possible to even a case the arrays used are not the same. Also, there is no limitation in data form or reorganizing the figure. Furthermore, comparison is possible beyond species, e.g. human and mouse.

The possibility of comparison beyond species shows that the invention is applicable to the field of pharmaceutical. For example, it is assumed that the substance conspicuously effective for the mouse is not efficacious for the human. Herein, by comparing between an array pattern of a certain organ of upon dosing the substance to a healthy mouse and an array pattern, due to a similar substance, in the same organ of a healthy person, primary screening of an analogous substance group is made possible.

## Claims

**1.** A data processing method for processing array data constituted by values representative of signal intensities on each spot arranged on a DNA chip by hybridization of the DNA chip to acquire data to be analyzed, the data processing method comprising:

a step of acquiring the array data;
a step of logarithmically converting the values representative of signal intensities on each spot constituting the array data; and
a step of generating converted data arranged with the logarithmically converted values correspondingly to the spot of the DNA chip.

**2.** A data processing method according to claim 1, further comprising

a step of scanning the logarithmically converted values to specify a median thereof, and
a step of subtracting the median from each value, thereby generating converted data comprising values the median has been subtracted.

**3.** A data processing method according to claim 1, further comprising a step of z-standardizing the logarithmically converted values to compute standardized values, thereby generating converted data comprising standardized values.

**4.** A data processing method according to any one of claims 1 to 3, further comprising a step of computing such a background value that a normal probability graph based on a cumulative frequency ratio of subtracted values obtained by subtracting a background value from each of the values representative of signal intensities has a predetermined linearity,

the values obtained by subtracting the background value from each of the values representative of signal intensities being rendered as a subject of logarithmic conversion.

**5.** A data processing method according to claim 4, wherein the step of computing a background value has

a step of specifying a minimum value of the values representative of signal intensities,
a step of setting a predetermined range including the minimum value,
a step of dividing the predetermined range by a predetermined number to compute, as background value candidates, an upper limit value, a lower limit value and a predetermined number of median values obtained by partitioning,
a step of subtracting, for each of the background value candidates, a background candidate value from each of the values representative of signal intensities to compute a subtracted value thereby determining a normal probability graph based on the subtracted values, and
a step of specifying a background candidate utilized in an excellent linearity of among the normal probability graph,

whereby a range of the upper limit value and lower limit value is changed to a satisfactory in a linearity concerning the specified background candidate, again repeating to compute a background value candidate, to compute a normal probability graph and to specify a background candidate.

**6.** A data processing method according to claim 4 or 5, wherein the step of representing the predetermined linearity carries out a chi-square test.

**7.** A data processing method according to claim 4, wherein the step of computing a background value has

a step of making reference to the values representative of signal intensities to specify values in a predetermined percentile of 2 or more, and
a step of deducing a background value on the basis of the specified values of 2 or more.

**8.** A data processing method according to claim 7, wherein the step of specifying values in the predetermined percentile includes

a step of making reference to the values representative of signal intensities to determine a lower quartile LQ, an upper quartile UQ and a median M,
the step of deducing a background value including a step of determining

$$\text{a background value } x = (UQ * LQ - M^2) / (UQ + LQ - 2M)$$

wherein, $x = 0$ when $UQ + DQ - 2M = 0$.

**9.** A data processing method according to any one of claims 1 to 8, further comprising

a step of classifying the spots into a plurality of groups according to an arrangement of the spots of the DNA chip,
a step of specifying, for each of the groups, from logarithmically converted values concerning the spot constituting the group, a median thereof, and
a step of subtracting the median from each of the logarithmically converted values.

**10.** A data processing method according to any one of claims 1 to 8, further comprising

a step of classifying the spots into a plurality of groups according to an arrangement of the spots of the DNA chip;
a step of specifying, for each of the groups, from values representative of signal intensities concerning spots constituting the group, a median thereof, and
a step of dividing each of the values representative of signal intensities by the median.

**11.** A data processing method according to claim 9 or 10, wherein the step of classification has a step of acquiring, based on each of one or a plurality of columns or one or a plurality of rows, logarithm values concerning the spots included in the column or row in the DNA chip.

**12.** A method of comparing values representative of signal intensities on a plurality of spots by utilizing a data processing method according to claim 2, the method **characterized by** having:

a step of dividing a converted data value related to one spot by a converted data value related to another spot.

**13.** A method of comparing values representative of signal intensities on a plurality of spots by utilizing a data processing method according to claim 3, the method **characterized by** having:

a step of computing a difference value between one standardized value and another standardized value.

**14.** A method according to claim 13, further comprising a step of computing an exponentiation of a predetermined number on the difference value.

**15.** A data processing program for a computer to execute a data processing method of processing array data constituted by values representative of signal intensities on each spot arranged on a DNA chip by hybridization of the DNA chip to acquire data to be analyzed, the data processing program for a computer to execute comprising:

a step of acquiring the array data;
a step of logarithmically converting the values representative of signal intensities on each spot constituting the array data; and
a step of generating converted data arranged with the logarithmically converted values correspondingly to the spot of the DNA chip.

**16.** A data processing program according to claim 15, wherein the computer is made to execute, further,

a step of scanning the logarithmically converted values to specify a median thereof, and

a step of subtracting the median from each value, thereby generating converted data comprising values the median has been subtracted.

**17.** A data processing program according to claim 16, wherein the computer is made to execute, further, a step of z-standardizing the logarithmically converted values to compute standardized values, thereby generating converted data comprising standardized values.

**18.** A data processing program according to any one of claims 15 to 17, wherein the computer is made to execute, further, a step of computing such a background value that a normal probability graph based on a cumulative frequency ratio of subtracted values obtained by subtracting a background value from each of the values representative of signal intensities has a predetermined linearity, the computer being operated such that the values obtained by subtracting a background value from each of the values representative of signal intensities is rendered as a subject of logarithmic conversion.

**19.** A data processing program according to claim 18, wherein the computer is made to execute, in the step of computing the background value,

a step of specifying a minimum value of the values representative of signal intensities,
a step of setting a predetermined range including the minimum value,
a step of partitioning the predetermined range by a predetermined number to compute, as background value candidates, an upper limit value, a lower limit value and a predetermined number of median values obtained by partitioning,
a step of subtracting, for each of the background value candidates, a background candidate value from each of the values representative of signal intensities to compute a subtracted value thereby determining a normal probability graph based on the subtracted values, and
a step of specifying a background candidate utilized in an excellent linearity of among the normal probability graph,

whereby a range of the upper limit value and lower limit value is changed to a satisfactory in a linearity concerning the specified background candidate, again for the computer to repeat to compute a background value candidate, to compute a normal probability graph and to specify a background candidate.

**20.** A data processing program according to claim 18 or 19,
wherein the computer is made to execute a chi-square test, in the step of representing the predetermined linearity.

**21.** A data processing program according to claim 18, wherein the computer is made to execute, in the step of computing a background value,

a step of making reference to the values representative of signal intensities to specify values in a predetermined percentile of 2 or more, and
a step of deducing a background value on the basis of the specified values of 2 or more.

**22.** A data processing program according to claim 21, wherein the computer is made to execute, in the step of computing a background value,

a step of determining a lower quartile LQ, an upper quartile UQ and a median M from the values representative of signal intensities,
the step of determining

$$x = (UQ * LQ - M^2) / (UQ + LQ - 2M)$$

wherein, x = 0 when UQ + DQ - 2M = 0 to take a determined x as a background value.

**23.** A data processing program according to any one of claims 14 to 22, wherein the computer is made to execute

a step of classifying the spots into a plurality of groups according to an arrangement of the spots of the DNA chip,
a step of specifying, for each of the groups, from logarithmically converted values concerning the spot constituting the group, a median thereof, and
a step of subtracting the median from each of the logarithmically converted values.

**24.** A data processing program according to any one of claims 15 to 22, wherein the computer is made to execute further

a step of classifying the spots into a plurality of groups according to a spot arrangement of the DNA chip;
a step of specifying, for each of the groups, from values representative of signal intensities concerning spots constituting the group, a median thereof, and
a step of dividing each of the values representative of signal intensities by the median.

**25.** A data processing program according to claim 24 or 25, wherein the computer is made to execute, in the step of classification, a step of acquiring, for each of one or a plurality of columns or one or a plurality of rows, logarithm values concerning the spots included in the column or row in the DNA chip.

**26.** A program for a computer to operate in order for comparing values representative of signal intensities on a plurality of spots,

the computer being made to execute a step constituting a data processing program according to claim 16, and
the computer being made to execute a step of dividing a converted data value related to one spot by a converted data value related to another spot.

**27.** A program for a computer to operate in order for comparing values representative of signal intensities on a plurality of spots, the program **characterized by**:

the computer being made to execute a step of constituting a data processing program according to claim 17, and
the computer being made to execute a step of computing a difference value between one standardized value and another standardized value.

**28.** A program according to claim 27, wherein the computer is made to further execute a step of computing an exponentiation of a predetermined number on the difference value.

FIG. 1

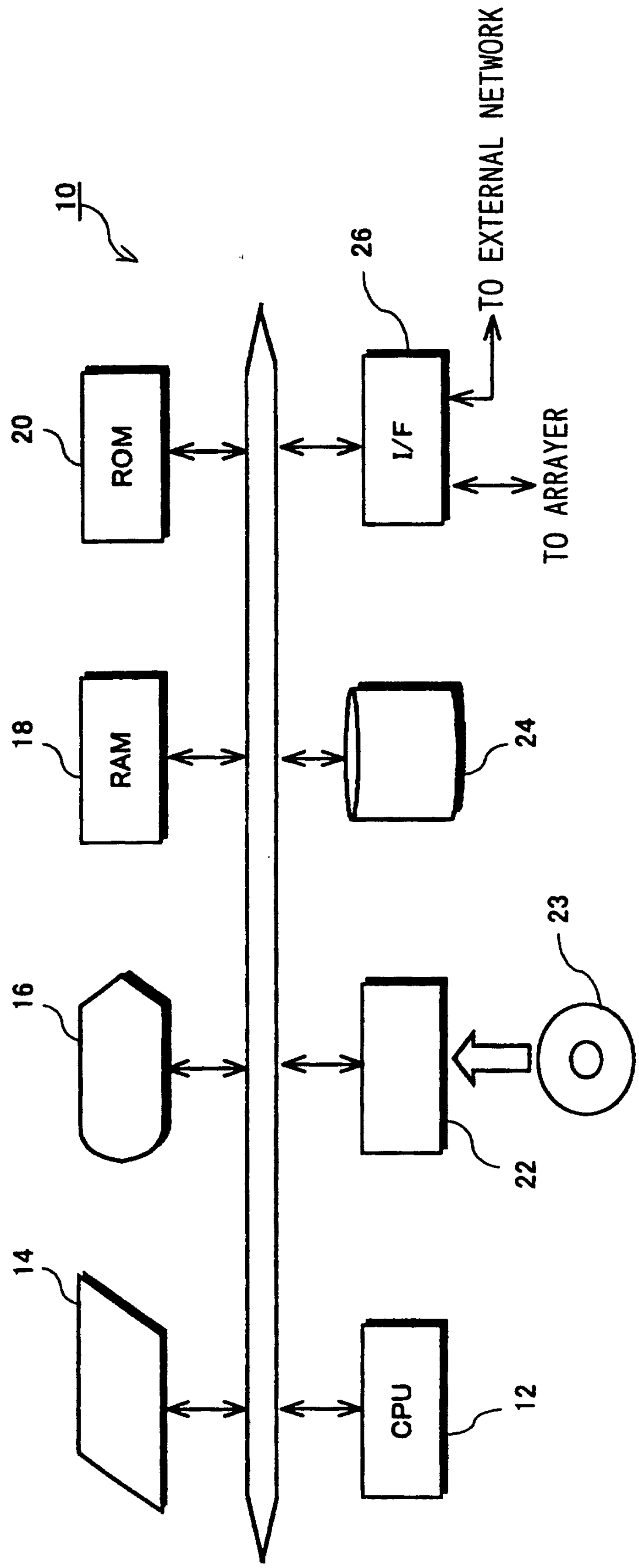
10
14
16
18
RAM
20
ROM
CPU
12
22
23
24
26
I/F
TO EXTERNAL NETWORK
TO ARRAYER

FIG. 2

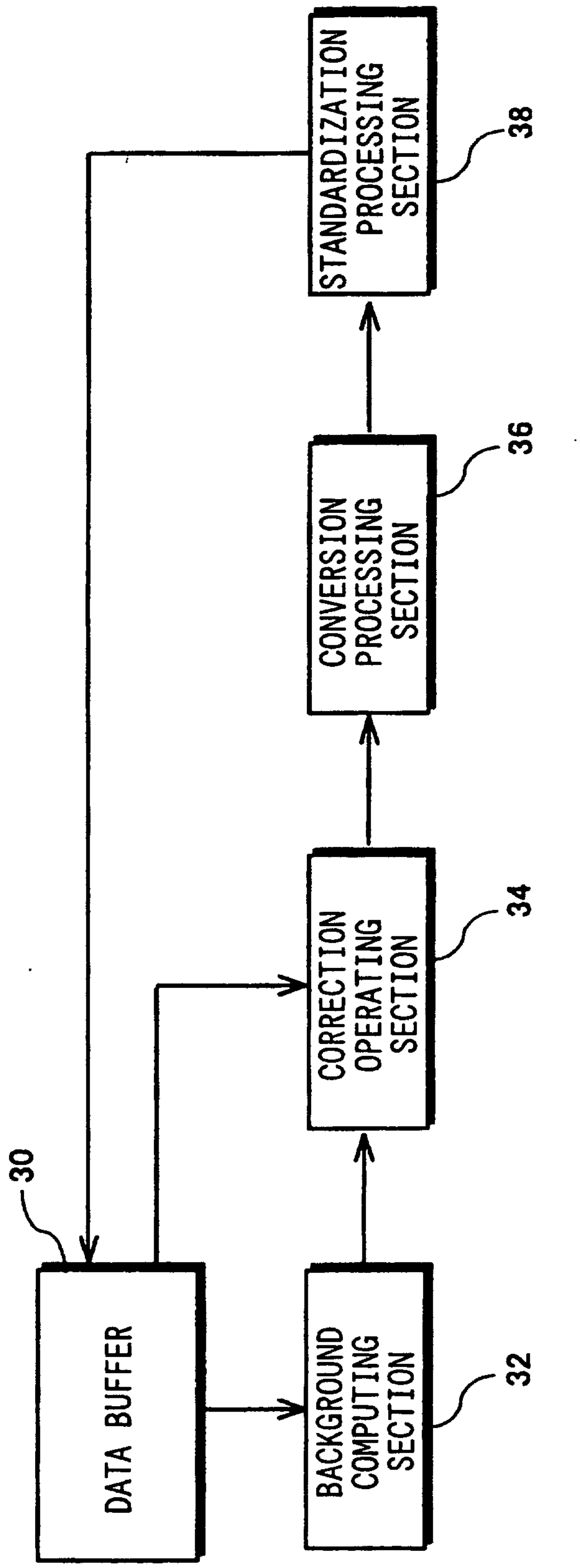
DATA BUFFER
30
BACKGROUND COMPUTING SECTION
32
CORRECTION OPERATING SECTION
34
CONVERSION PROCESSING SECTION
36
STANDARDIZATION PROCESSING SECTION
38

FIG. 3

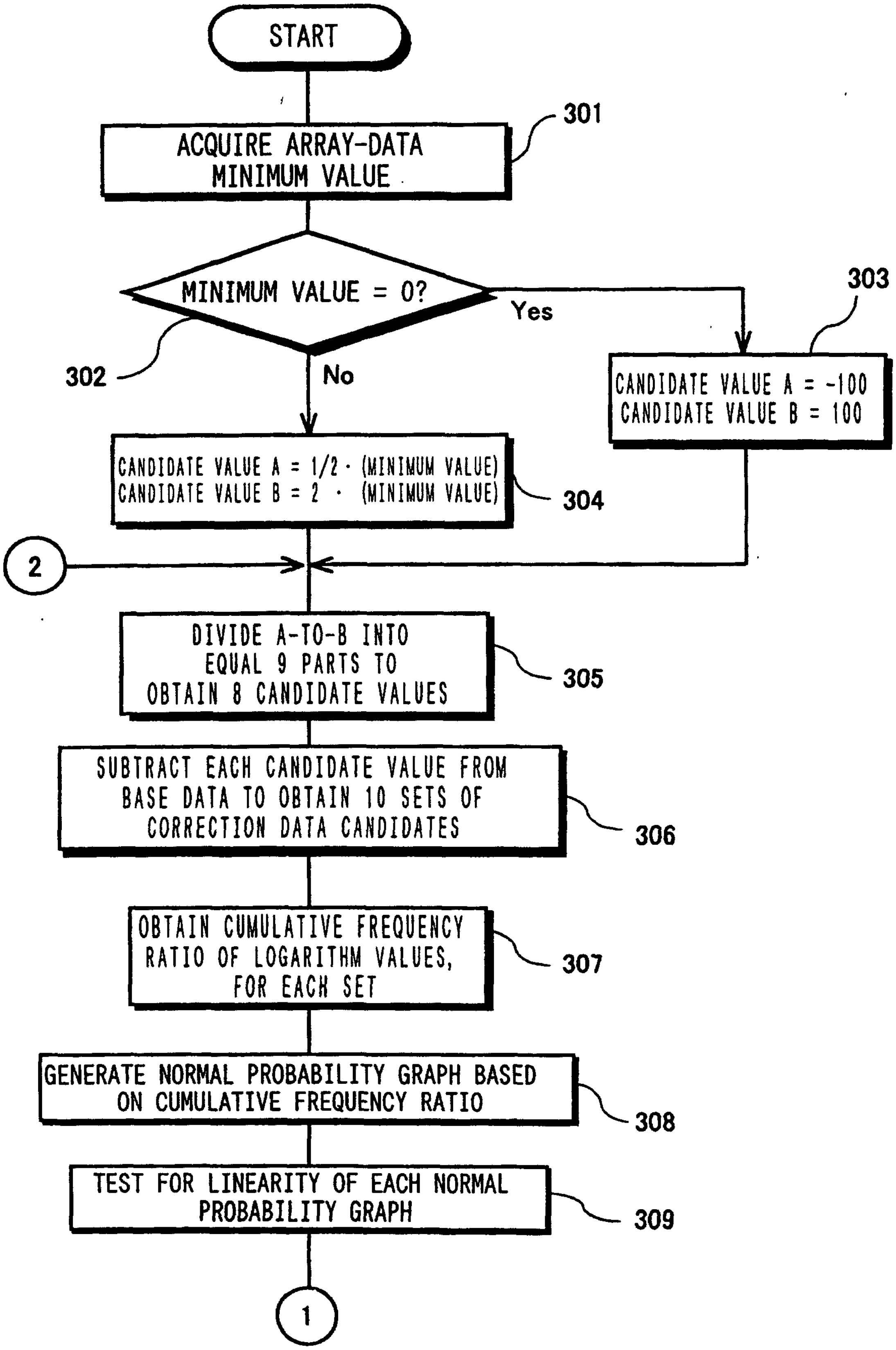
START
ACQUIRE ARRAY-DATA MINIMUM VALUE
301
MINIMUM VALUE = 0?
302
Yes
No
303
CANDIDATE VALUE A = -100
CANDIDATE VALUE B = 100
CANDIDATE VALUE A = 1/2 · (MINIMUM VALUE)
CANDIDATE VALUE B = 2 · (MINIMUM VALUE)
304
2
DIVIDE A-TO-B INTO EQUAL 9 PARTS TO OBTAIN 8 CANDIDATE VALUES
305
SUBTRACT EACH CANDIDATE VALUE FROM BASE DATA TO OBTAIN 10 SETS OF CORRECTION DATA CANDIDATES
306
OBTAIN CUMULATIVE FREQUENCY RATIO OF LOGARITHM VALUES, FOR EACH SET
307
GENERATE NORMAL PROBABILITY GRAPH BASED ON CUMULATIVE FREQUENCY RATIO
308
TEST FOR LINEARITY OF EACH NORMAL PROBABILITY GRAPH
309
1

FIG. 4

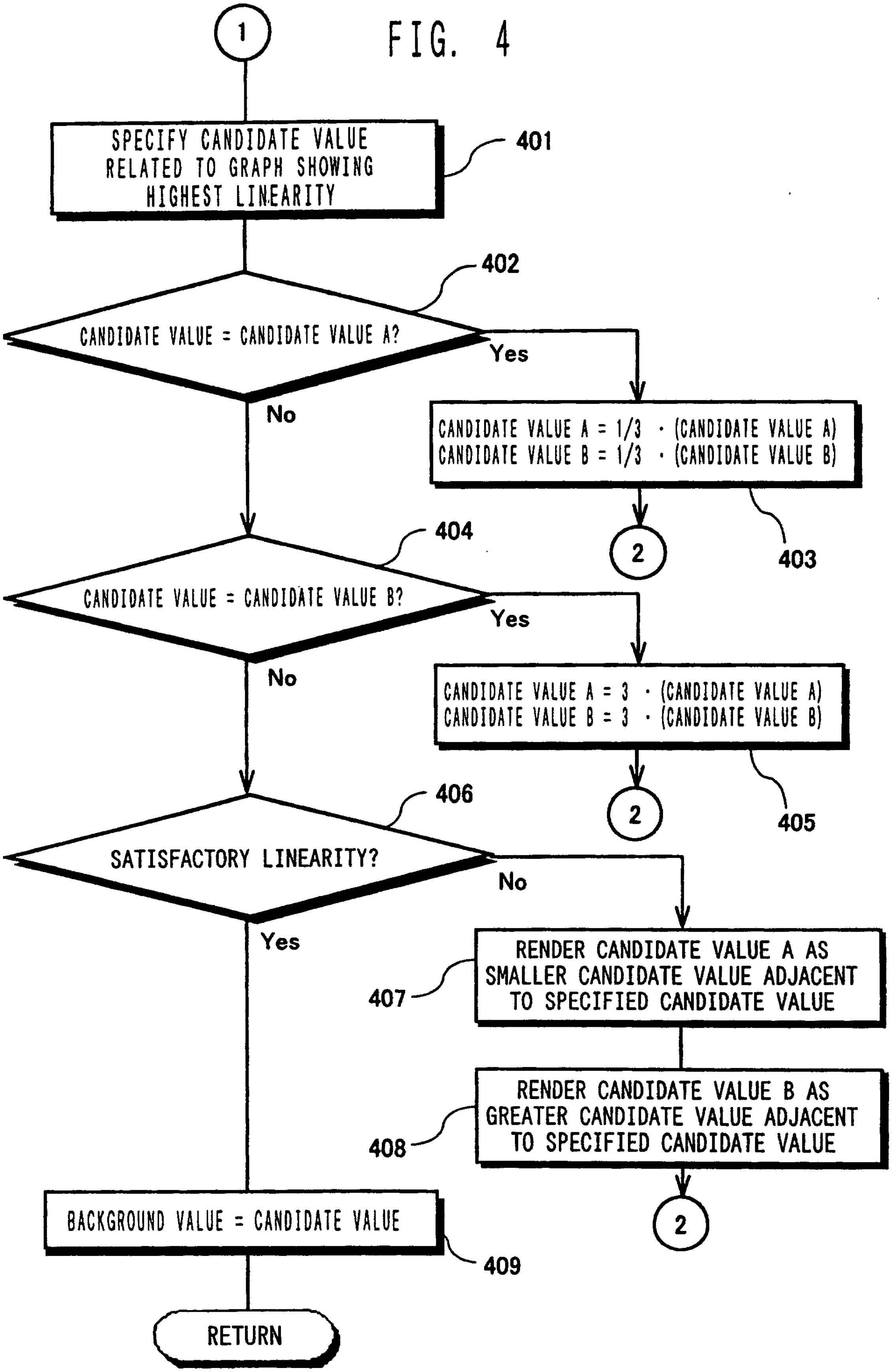
1
SPECIFY CANDIDATE VALUE RELATED TO GRAPH SHOWING HIGHEST LINEARITY
401
402
CANDIDATE VALUE = CANDIDATE VALUE A?
Yes
No
CANDIDATE VALUE A = 1/3 · (CANDIDATE VALUE A)
CANDIDATE VALUE B = 1/3 · (CANDIDATE VALUE B)
403
2
404
CANDIDATE VALUE = CANDIDATE VALUE B?
Yes
No
CANDIDATE VALUE A = 3 · (CANDIDATE VALUE A)
CANDIDATE VALUE B = 3 · (CANDIDATE VALUE B)
405
2
406
SATISFACTORY LINEARITY?
No
Yes
RENDER CANDIDATE VALUE A AS SMALLER CANDIDATE VALUE ADJACENT TO SPECIFIED CANDIDATE VALUE
407
RENDER CANDIDATE VALUE B AS GREATER CANDIDATE VALUE ADJACENT TO SPECIFIED CANDIDATE VALUE
408
2
BACKGROUND VALUE = CANDIDATE VALUE
409
RETURN

FIG. 5A

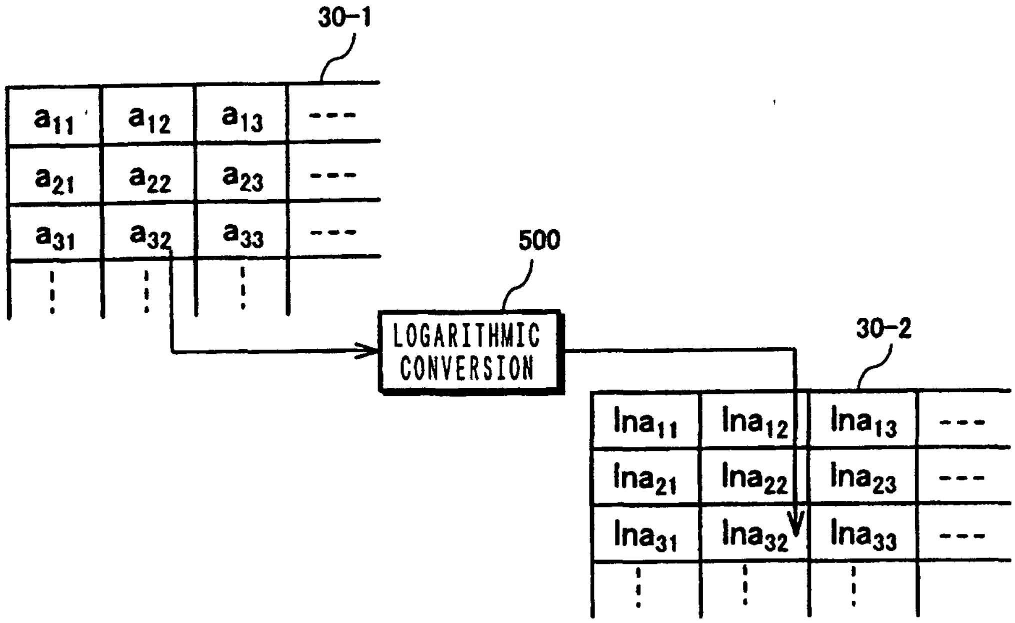
30-1
a11
a12
a13
a21
a22
a23
a31
a32
a33
500
LOGARITHMIC CONVERSION
30-2
lna11
lna12
lna13
lna21
lna22
lna23
lna31
lna32
lna33

FIG. 5B

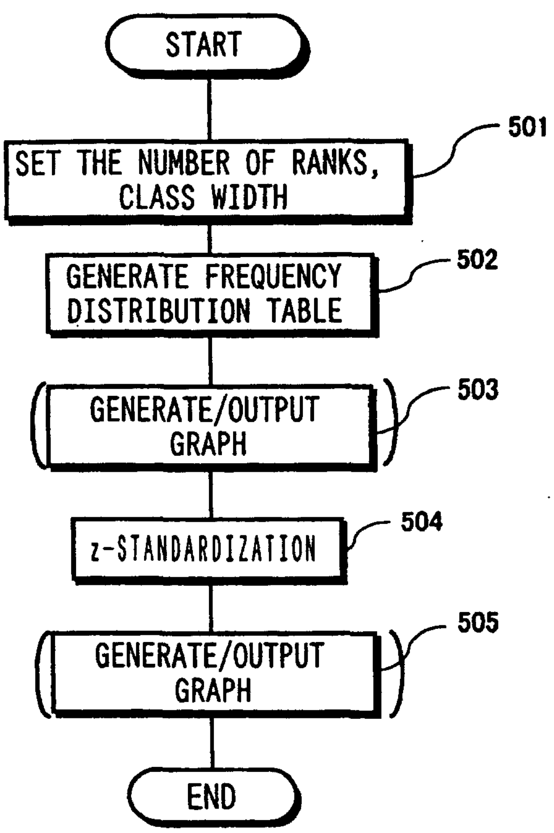
START
SET THE NUMBER OF RANKS, CLASS WIDTH
501
GENERATE FREQUENCY DISTRIBUTION TABLE
502
GENERATE/OUTPUT GRAPH
503
z-STANDARDIZATION
504
GENERATE/OUTPUT GRAPH
505
END

FIG. 6

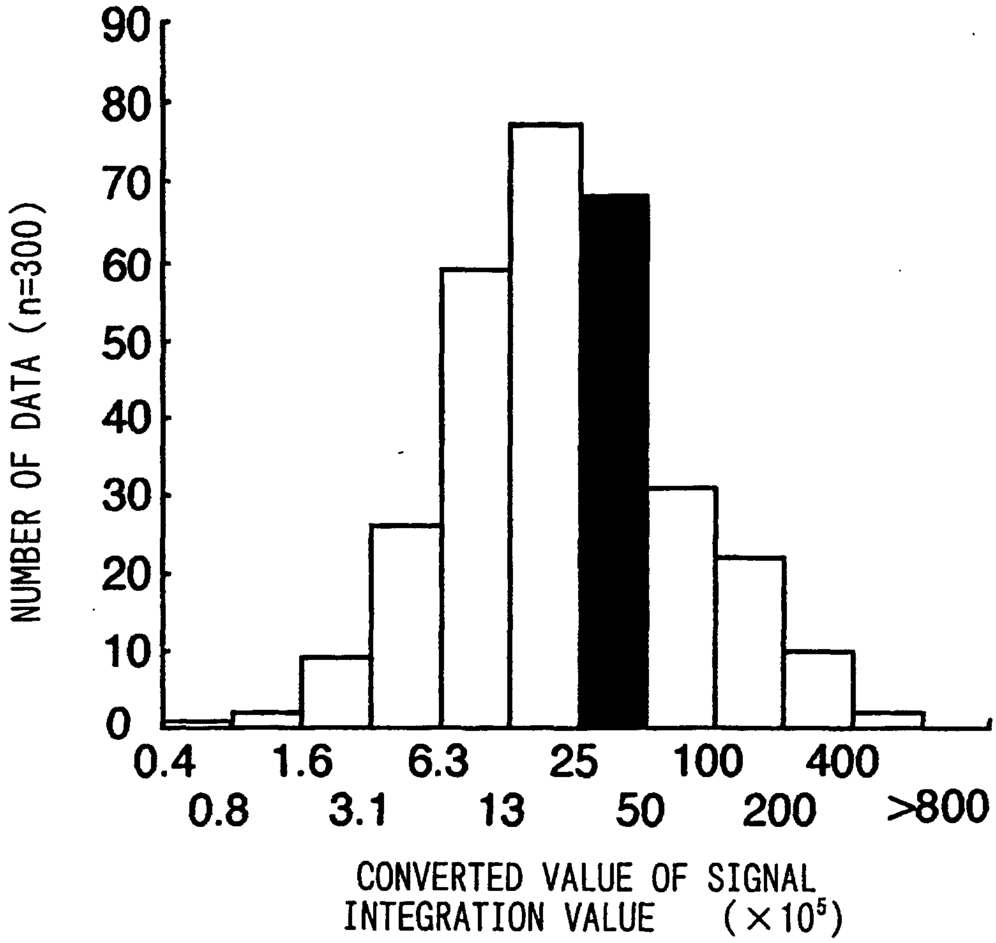
90
80
70
60
50
40
30
20
10
0
NUMBER OF DATA (n=300)
0.4
0.8
1.6
3.1
6.3
13
25
50
100
200
400
>800
CONVERTED VALUE OF SIGNAL
INTEGRATION VALUE (×10⁵)

FIG. 7

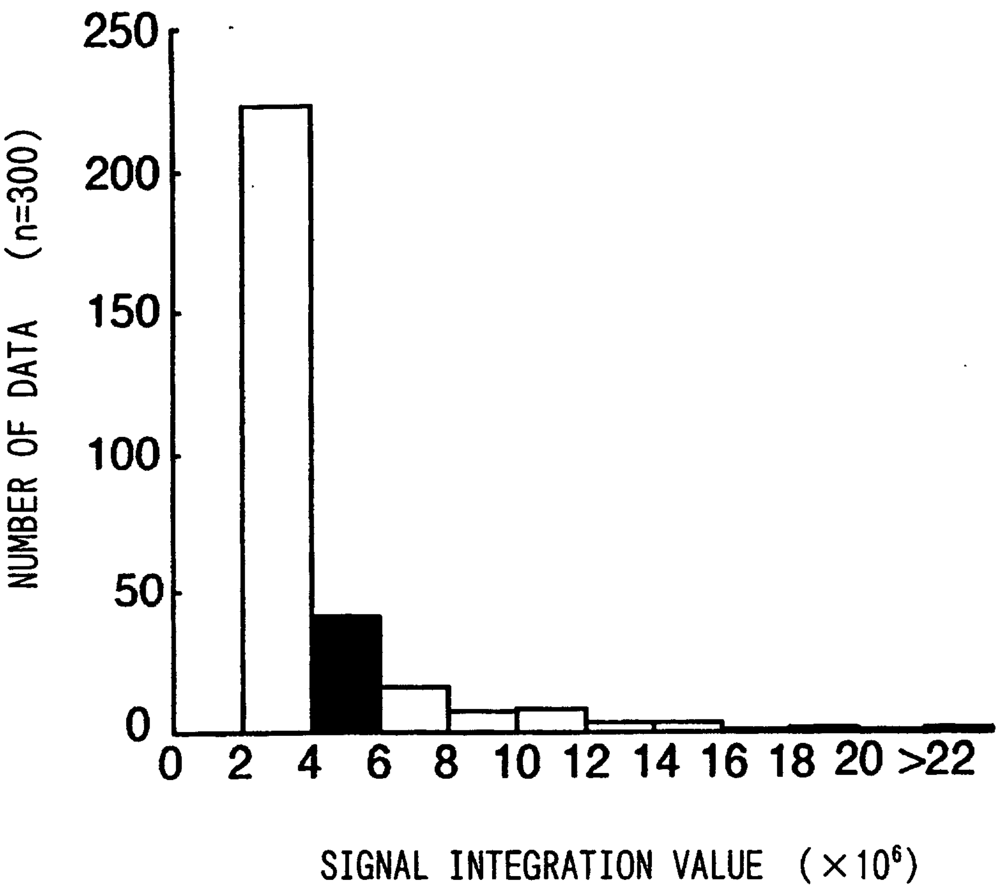
NUMBER OF DATA (n=300)
250
200
150
100
50
0
0 2 4 6 8 10 12 14 16 18 20 >22
SIGNAL INTEGRATION VALUE (×10⁶)

FIG. 8

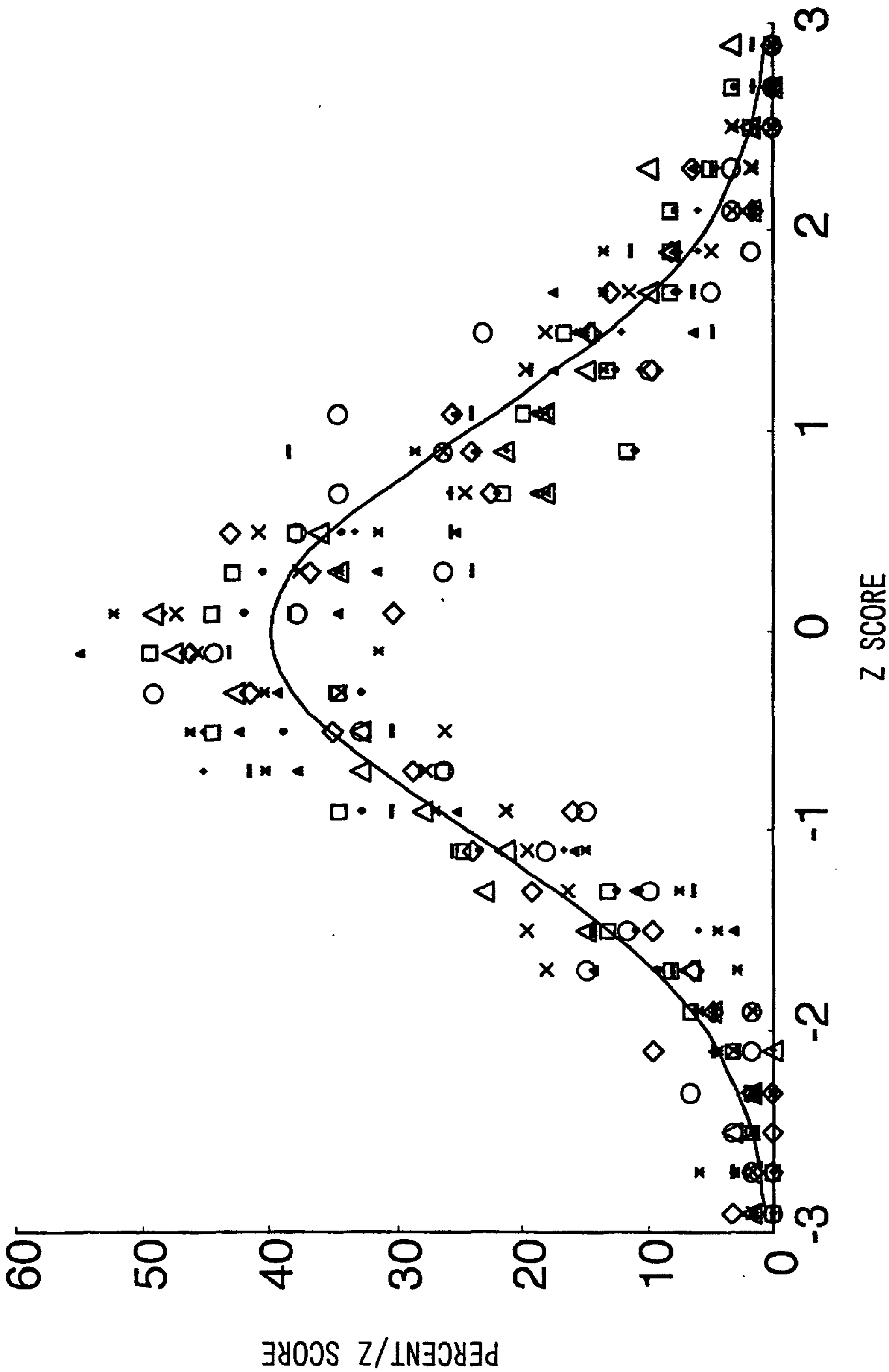

FIG. 9

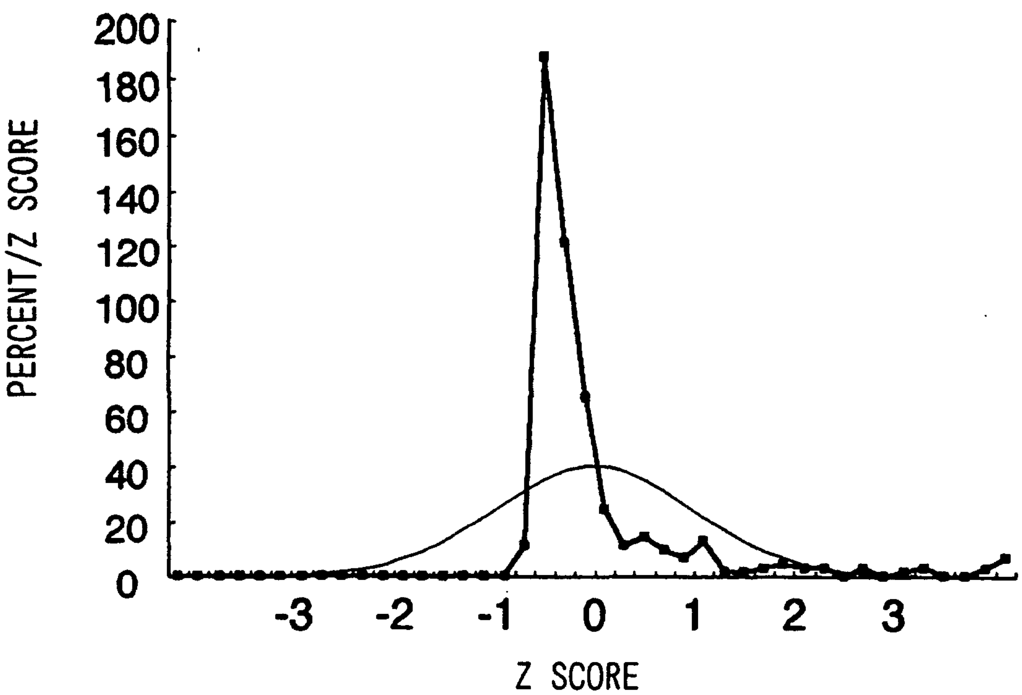
200
180
160
140
120
100
80
60
40
20
0
PERCENT/Z SCORE
-3
-2
-1
0
1
2
3
Z SCORE

FIG. 10A

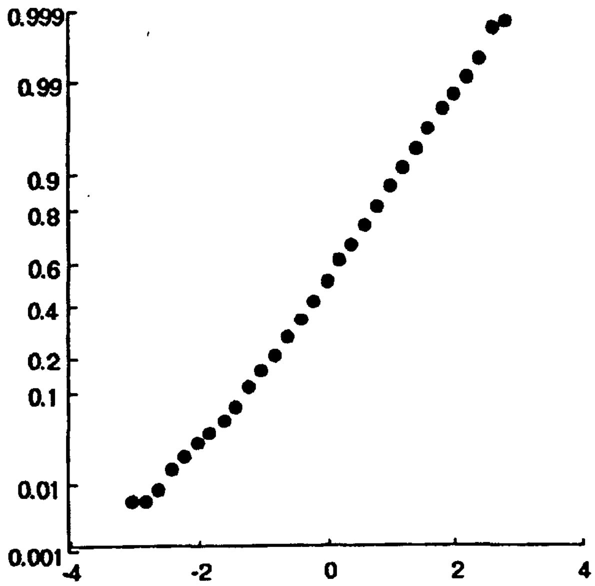


FIG. 10B

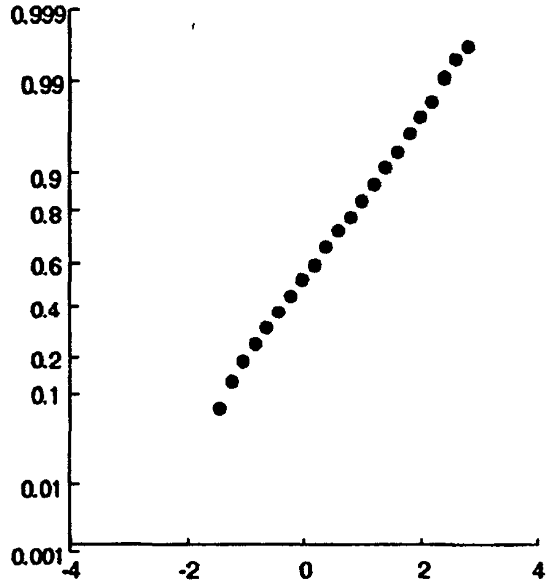


FIG. 10C

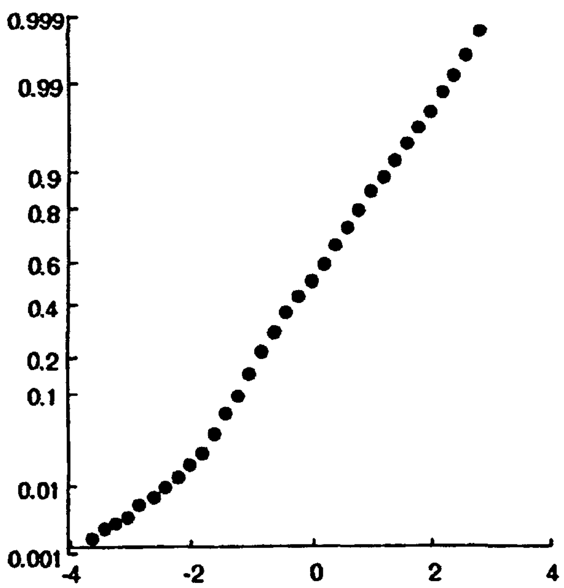


FIG. 10D

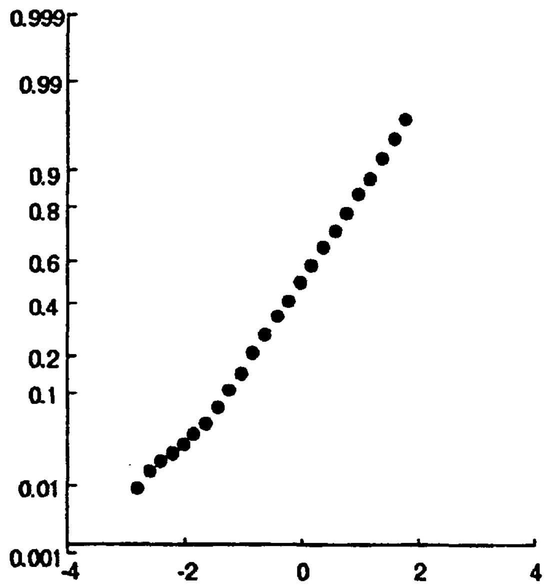

FIG. 11A

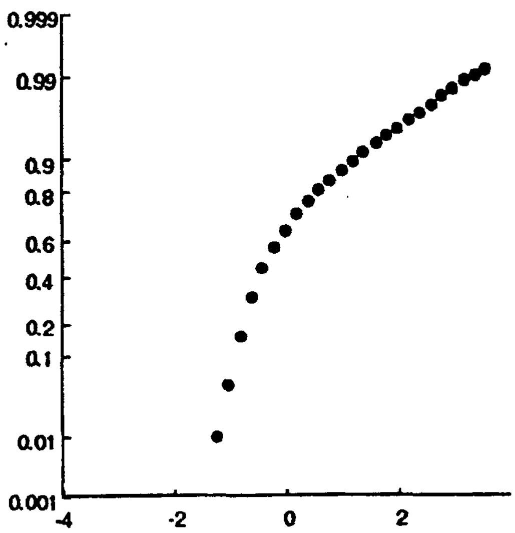


FIG. 11B

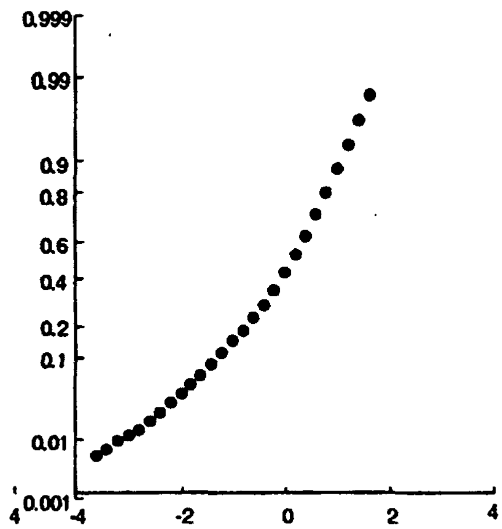


FIG. 11C

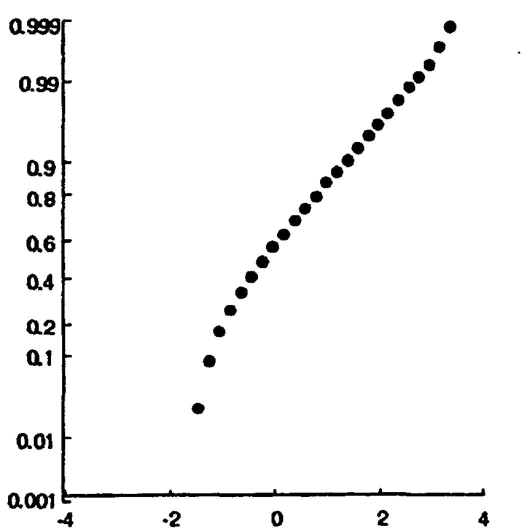


FIG. 11D

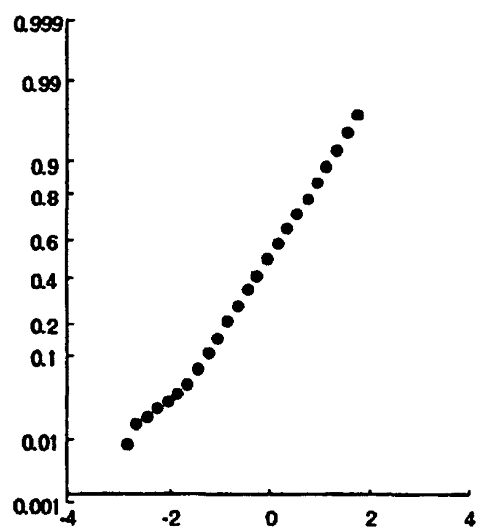

FIG. 12A

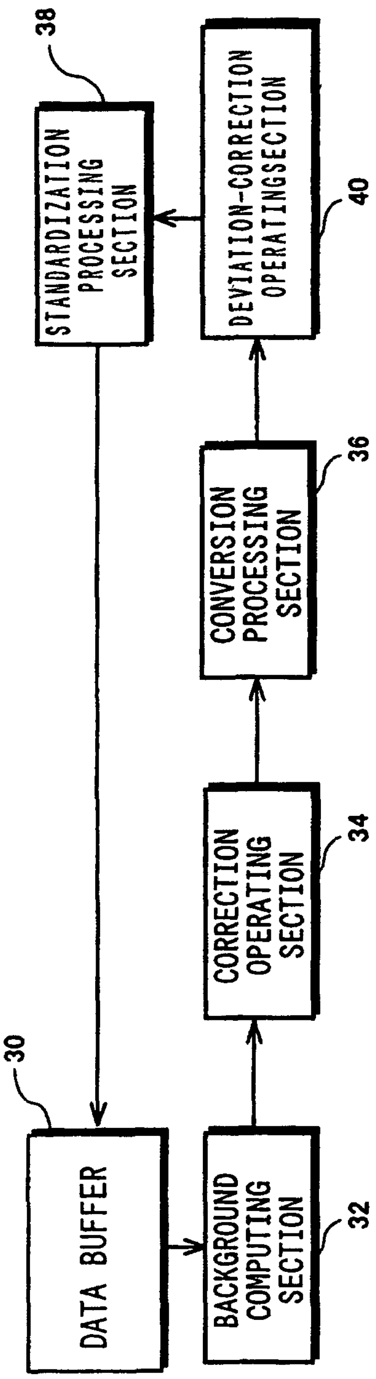


FIG. 12B

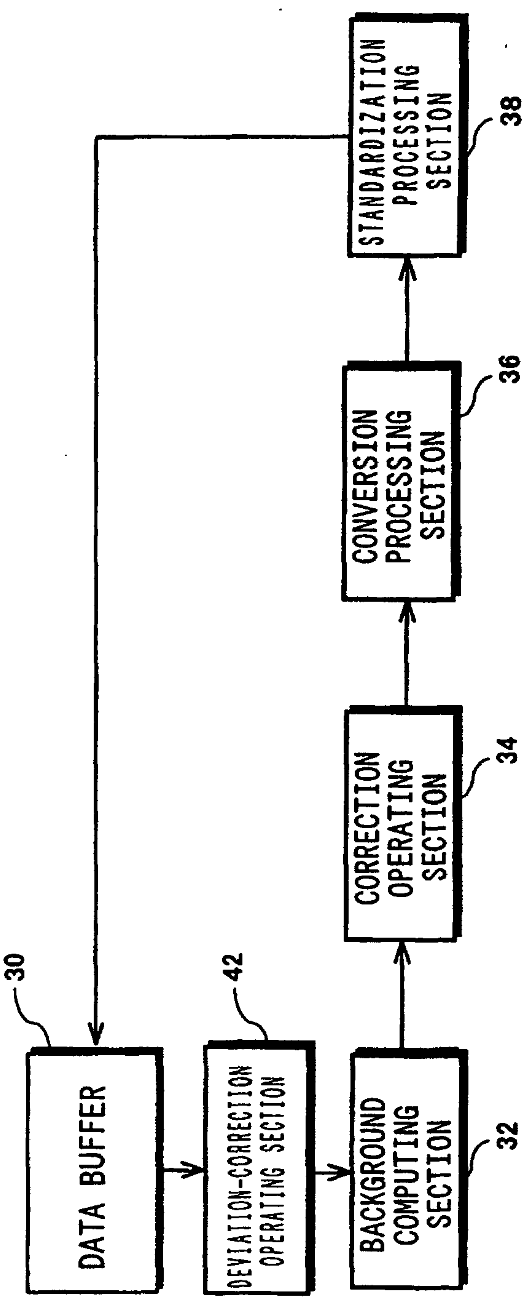

FIG. 13

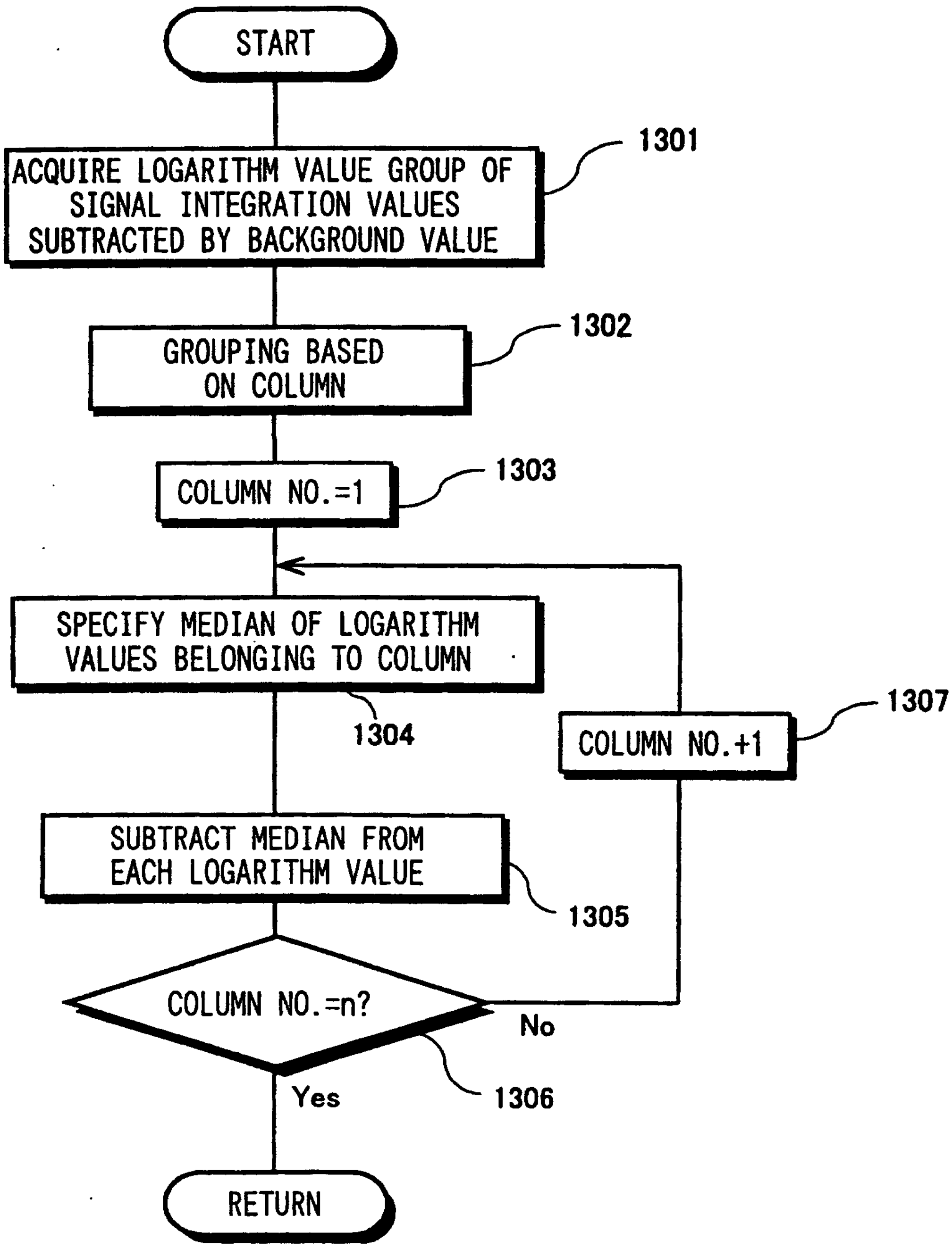
START
ACQUIRE LOGARITHM VALUE GROUP OF SIGNAL INTEGRATION VALUES SUBTRACTED BY BACKGROUND VALUE
1301
GROUPING BASED ON COLUMN
1302
COLUMN NO.=1
1303
SPECIFY MEDIAN OF LOGARITHM VALUES BELONGING TO COLUMN
1304
COLUMN NO.+1
1307
SUBTRACT MEDIAN FROM EACH LOGARITHM VALUE
1305
COLUMN NO.=n?
No
1306
Yes
RETURN

FIG. 14

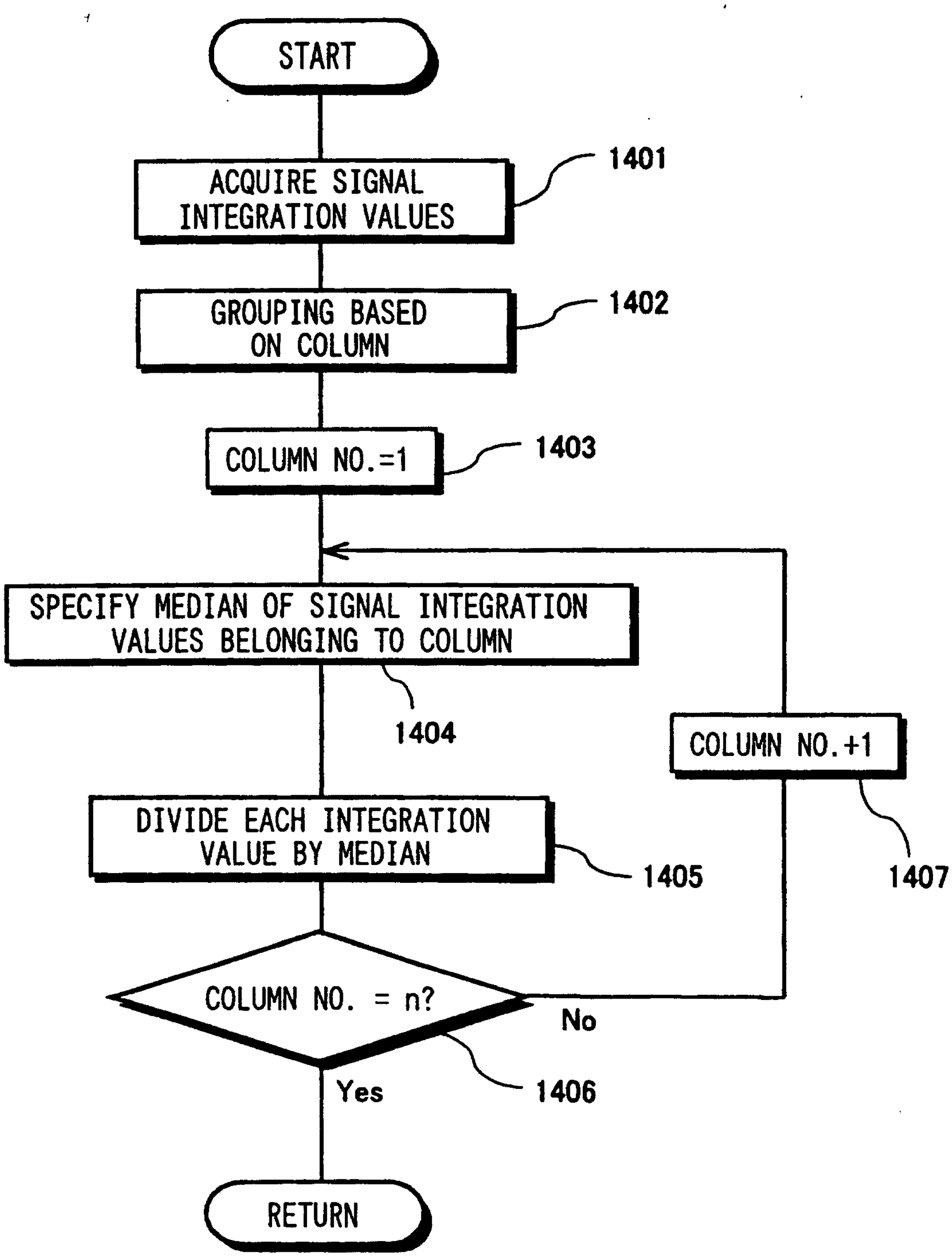
START
ACQUIRE SIGNAL INTEGRATION VALUES
1401
GROUPING BASED ON COLUMN
1402
COLUMN NO.=1
1403
SPECIFY MEDIAN OF SIGNAL INTEGRATION VALUES BELONGING TO COLUMN
1404
COLUMN NO.+1
1407
DIVIDE EACH INTEGRATION VALUE BY MEDIAN
1405
COLUMN NO. = n?
No
Yes
1406
RETURN

FIG. 15A

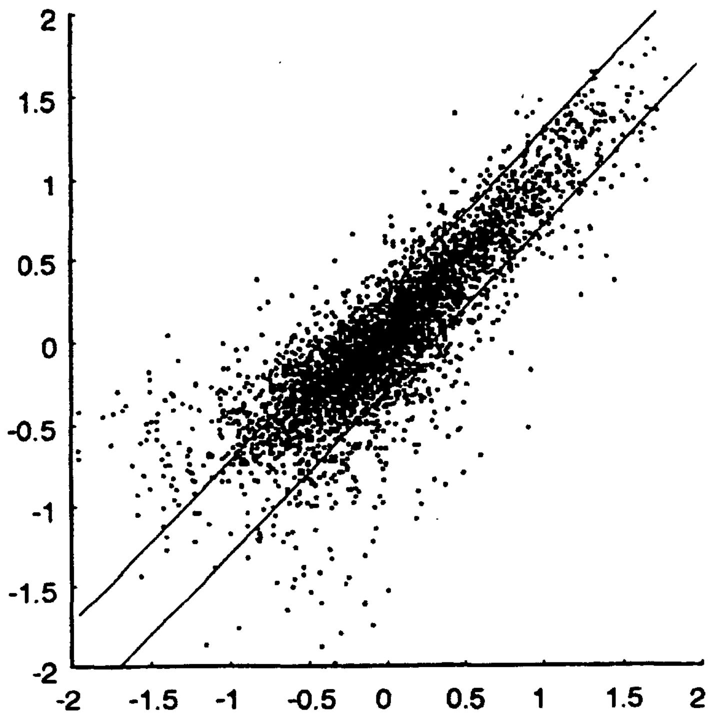


FIG. 15B

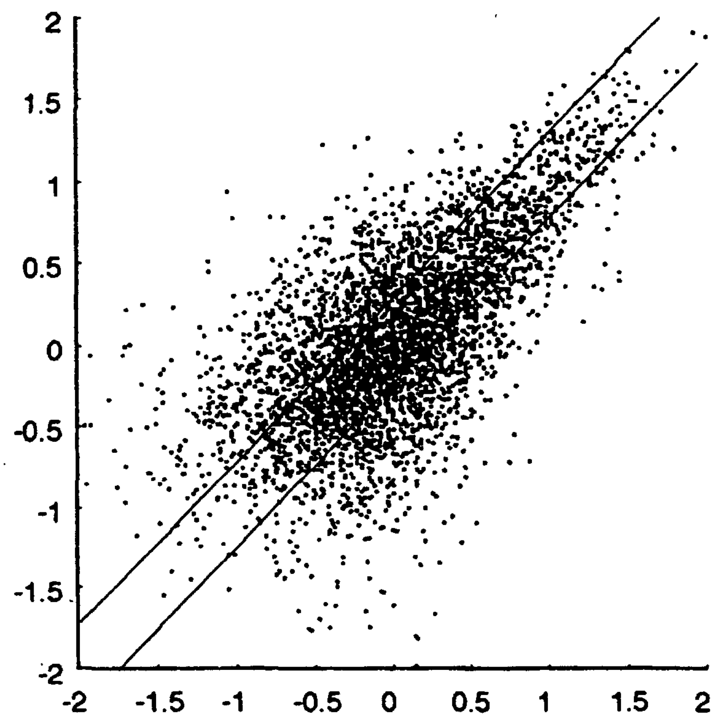

## INTERNATIONAL SEARCH REPORT

International application No.
PCT/JP01/04697

A. CLASSIFICATION OF SUBJECT MATTER
Int.Cl$^7$ G06F19/00, C12N15/00

According to International Patent Classification (IPC) or to both national classification and IPC

B. FIELDS SEARCHED

Minimum documentation searched (classification system followed by classification symbols)
Int.Cl$^7$ G06F19/00, C12N15/00

Documentation searched other than minimum documentation to the extent that such documents are included in the fields searched
Jitsuyo Shinan Koho 1922-1996 Toroku Jitsuyo Shinan Koho 1994-2001
Kokai Jitsuyo Shinan Koho 1971-2001 Jitsuyo Shinan Toroku Koho 1996-2001

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| X | Schuchhardt, J. et al., "Normalization strategies for cDNA microarrays", Nucleic Acids Research, May 15, 2000, Vol.28, No.10, page e47 (i-v) | 1-3,15-17 |
| A | Schuchhardt, J. et al., "Normalization strategies for cDNA microarrays", Nucleic Acids Research, May 15, 2000, Vol.28, No.10, page e47 (i-v) | 4-14,18-28 |

☐ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 18 July, 2001 (18.07.01) | 07 August, 2001 (07.08.01) |
| Name and mailing address of the ISA/ Japanese Patent Office | Authorized officer |
| Facsimile No. | Telephone No. |

Form PCT/ISA/210 (second sheet) (July 1992)